# FASCICULE DE BREVET EUROPEEN

(11) **EP 2 429 659 B1**
(45) Date de publication et mention de la délivrance du brevet: **13.12.2017**
(21) Numéro de dépôt: 10723643.2
(22) Date de dépôt: 12.05.2010
(51) Int. Cl.: A61P 31/04, A61K 39/095, A61K 9/127, A61K 39/02, A61K 39/10

(54) **PROCÉDÉ DE DÉTOXIFICATION DU LIPOPOLYSACCHARIDE (LPS) OU DU LIPIDE A DES BACTÉRIES À GRAM-NÉGATIF**
VERFAHREN ZUR ENTGIFTUNG DES LIPOPOLYSACCHARIDS (LPS) ODER VON LIPID A VON GRAM-NEGATIVEN BAKTERIEN
METHOD FOR DETOXIFICATION OF LIPOPOLYSACCHARIDE (LPS) OR OF LIPID A OF GRAM-NEGATIVE BACTERIA

(30) Priorité: 14.05.2009 FR 0902331; 29.07.2009 US 271985 P
(43) Date de publication de la demande: 21.03.2012
(73) Titulaire: Sanofi Pasteur, 69367 Lyon Cedex 07 (FR)
(72) Inventeur: HAENSLER, Jean, 69290 Grezieu la Varenne (FR); DALENCON, François, 69004 Lyon (FR); MOREAU, Monique, 69007 Lyon (FR); MISTRETTA, Noëlle, 69210 Sain Bel (FR)
(86) Numéro de dépôt international: PCT/FR2010/000366
(87) Numéro de publication internationale: WO 2010/130897

(56) Documents cités:
- WO-A2-2005/060330
- FR-A1- 2 862 306
- PETROV A B ET AL: "TOXICITY AND IMMUNOGENICITY OF NEISSERIA-MENINGITIDIS LIPOPOLYSACCHARIDE INCORPORATED INTO LIPOSOMES" INFECTION AND IMMUNITY, vol. 60, no. 9, 1992, pages 3897-3903, XP002558878 ISSN: 0019-9567
- YAN W ET AL: "Mechanism of adjuvant activity of cationic liposome: Phosphorylation of a MAP kinase, ERK and induction of chemokines" MOLECULAR IMMUNOLOGY, PERGAMON, GB, vol. 44, no. 15, 1 juillet 2007 (2007-07-01), pages 3672-3681, XP025320886 ISSN: 0161-5890 [extrait le 2007-05-30]
- LONEZ C ET AL: "Cationic liposomal lipids: From gene carriers to cell signaling" PROGRESS IN LIPID RESEARCH, PERGAMON PRESS, PARIS, FR, vol. 47, no. 5, 1 septembre 2008 (2008-09-01), pages 340-347, XP022715976 ISSN: 0163-7827 [extrait le 2008-04-01]
- SPOHN R ET AL: "Synthetic lipopeptide adjuvants and Toll-like receptor 2-structure-activity relationships" VACCINE, BUTTERWORTH SCIENTIFIC. GUILDFORD, GB, vol. 22, no. 19, 23 juin 2004 (2004-06-23) , pages 2494-2499, XP004515469 ISSN: 0264-410X
- J Dijkstra ET AL: "Incorporation of LPS in liposomes diminishes its ability to induce tumoricidal activity and tumor necrosis factor secretion in murine macrophages", Journal of Leukocyte Biology, 1 May 1988 (1988-05-01), pages 436-444, XP055115734, UNITED STATES Retrieved from the Internet: URL:http://www.jleukbio.org/content/43/5/4 36.abstract [retrieved on 2017-06-01]
- CHRISTENSEN DENNIS ET AL: "Cationic liposomes as vaccine adjuvants", EXPERT REVIEW OF VACCINES, FUTURE DRUGS, LONDON, GB, vol. 6, no. 5, 1 October 2007 (2007-10-01) , pages 785-796, XP008137314, ISSN: 1476-0584, DOI: 10.1586/14760584.6.5.785
- "Productinformation 1,2-Diacyl-sn-glycero-3-phosphocholine", Sigma website , pages 1-1, Retrieved from the Internet: URL:https://www.sigmaaldrich.com/content/d am/sigma-aldrich/docs/Sigma/Product_Inform ation_Sheet/1/p3556pis.pdf [retrieved on 2014-11-07]

## Description

L'invention s'inscrit dans le domaine des vaccins et a pour objet un procédé permettant de détoxifier le lipopolysaccharide (LPS) ou le lipide A des bactéries à Gram-négatif qui peut être par la suite utilisé dans les vaccins à titre d'adjuvant et /ou d'antigène vaccinal.

Le LPS est un constituant majeur de la membrane externe de la paroi des bactéries à Gram-négatif. Le LPS est toxique à forte dose pour les mammifères et au regard de cette activité biologique, a été dénommé endotoxine. Il est responsable du choc septique, pathologie mortelle qui se développe suite à une infection aiguë par une bactérie à Gram-négatif

La structure du LPS est constituée d'une partie lipidique, appelée le lipide A, liée de manière covalente à une partie polysaccharidique.

Le lipide A est responsable de la toxicité du LPS. Il est fortement hydrophobe et permet d'ancrer le LPS dans la membrane externe de la paroi. Le lipide A est composé d'une structure disaccharidique substituée par des chaînes d'acides gras. Le nombre et la composition des chaînes d'acides gras varient d'une espèce à l'autre.

La partie polysaccharidique est constituée par des chaînes de carbohydrates qui sont responsables de l'antigénicité. On distingue au moins 3 grandes régions dans cette partie polysaccharidique :
(i) un core interne, constitué de monosaccharides [un ou plusieurs KDO (acide 2-céto-3-desoxy-octulosonique) et un ou plusieurs heptose (Hep)] invariants au sein d'une même espèce bactérienne ;
(ii) un core externe lié à un heptose et constitué de divers monosaccharides ; et
(iii) une chaîne externe O-spécifique constituée d'un enchaînement d'unités répétitives - unités répétitives elles-mêmes composées de un ou plusieurs monosaccharides différents.

La composition de la partie polysaccharidique varie d'une espèce à une autre, d'un sérotype (immunotype chez le méningoccoque) à un autre au sein d'une même espèce.

Chez un certain nombre de bactéries non-entériques à Gram-négatif telles que les *Neisseriae, Bordetellae, Branhamellas, Haemophilus* et *Moraxellae,* la chaîne O-spécifique n'existe pas. La partie saccharidique de LPS de ces bactéries n'est constituée que du core oligosaccharidique). Par conséquent, le LPS de ces bactéries est souvent dénommé lipooligosaccharide (LOS).

Le LPS n'est pas seulement toxique, il est aussi immunogène. Chez les mammifères, les anticorps anti-LPS sont générés pendant le portage et l'infection et peuvent être protecteurs. Ainsi, l'usage du LPS a déjà été envisagé dans la prophylaxie des infections dues aux bactéries à Gram-négatif et des maladies associées. De plus quand il est associé avec un autre antigène d'intérêt, il peut être aussi capable d'avoir un effet adjuvant - c'est-à-dire d'accroire la réponse immune d'un mammifère à l'encontre de cet antigène vaccinal.

Néanmoins, il convient au préalable de le détoxifier. Pour ce faire, il n'est pas impératif de retirer le lipide A dans son intégralité. En effet, la toxicité étant plus particulièrement liée à une conformation supra moléculaire conférée par la totalité des chaînes d'acides gras portées par le noyau disaccharidique du lipide A, il suffit, selon un mode avantageux, d'agir au niveau de ces chaînes. La détoxification peut être obtenue selon des approches diverses : chimique, enzymatique, génétique ou bien par complexation avec un peptide analogue de la polymixine B, ou bien encore en associant le LPS à des lipides de manière à former des complexes tels que des liposomes. En effet, le LPS ou le lipide A en liposomes - c'est-à-dire associé à la bicouche lipidique formant les liposomes - peut voir sa toxicité décroître de manière très substantielle. Le ou les lipides entrant dans la composition de ces complexes lipidiques *i.a.* de ces liposomes, peuvent être des lipides neutres, cationic et/ou anioniques. Ceci est décrit dans (i) Petrov et al, Infect. Immun. (1992) 60 (9) : 3897 qui utilise un mélange de lipides neutres, la phosphatidylcholine et le cholestérol ; (ii) Richards et al, Vaccine (1989) 7 : 506 qui utilise un mélange de lipides neutre (dimyristoyl phosphatidylcholine, cholestérol) et anionique (dicetyl phosphate, dimyristoyl phosphatidylglycerol) ; et (iii) Bennett-Guerrero et al, Infect. Immun. (2000) 68 (11) : 6202 qui utilise un mélange de lipides neutres (dimysristoyl phosphatidyl choline et cholestérol) et anionique (dimysristoyl phosphatidylglycérol) ; et (iv) Tseng et al, Vet. Immunol. Immunopath. (2009) 131 : 285 qui utilise un mélange particulier de cholesterol, de stearyl amine de 1,2-di-palmitoyl-sn-glycero-3-phospho-L-serine (DPPC) avec en final, la production de liposomes cationiques.

Au cours d'études comparatives on a maintenant trouvé que des liposomes cationiques possédaient un pouvoir détoxifiant supérieur à celui des liposomes neutres ou anioniques. Les tests utilisés à des fins de comparaison sont aux nombre de deux :
- Le test pyrogène chez le lapin. Ce test, les calculs et leur lecture et ont été réalisés selon les principes énoncés par la Pharmacopée Européenne (Edition 6.0, paragraph 2.6.8.).
- Le test LAL (Limulus Amebocyte Lysate) réalisé selon les principes énoncés par la Pharmacopée Européenne (Edition 6.0, paragraph 2.6.14.).

C'est pourquoi, l'invention a pour objet un procédé de détoxification du lipopolysaccharide (LPS) ou du lipide A d'une bactérie à Gram-négatif selon lequel on met en mélange le LPS ou le lipide A avec un lipide cationique pour former un complexe dans lequel le LPS est associé au lipide cationique.

L'invention a pour objet un procédé de détoxification du lipopolysaccharide (LPS) ou du lipide A d'une bactérie à Gram-négatif selon lequel on mélange le LPS ou le lipide A avec un lipide cationique pour former un complexe dans lequel le LPS est associé au lipide cationique, le lipide cationique étant sous forme de liposomes cationiques, et le LPS ou le lipide A étant incorporé aux liposomes, et le lipide cationique étant sélectionné dans le groupe constitué par (i) les lipides incorporant une structure éthyiphosphochotine : et (ii) les dérivés cationiques du cholestérol.

Il est décrit un complexe comprenant au moins le LPS ou le lipide A d'une bactérie à Gram-négatif et un lipide cationique dans lequel le LPS ou le lipide A est détoxifié en raison de sa complexation au lipide cationique.

### Le LPS / lipide A

Le LPS / lipide A pouvant être détoxifié par le procédé selon l'invention peut être n'importe quel LPS / lipide A de bactérie à Gram-négatif, qu'elles soient entériques ou non-entériques, de préférence pathogènes. Sous un aspect particulier, il peut s'agir du LPS / lipide A de bactéries non-entériques des genres tels que les *Neisseriae, Bordetellae, Branhamellas, Haemophilus* et *Moraxellae.* Le LPS de ces bactéries est aussi désigné sous le terme de LOS (lipooligosaccharide) en raison de l'absence de polysaccharide O-spécifique. A titre d'exemple additionnel, on cite le LPS/LOS des genres ou espèces *Klebsiella, Pseudomonas, Burkolderia, Porphyromonas, Franciscella, Yersinia, Enterobacter, Salmonella, Shigella, E. coli ;* et tout particulièrement le LOS de *N. meningitidis.*

Les souches de *N. meningitidis* sont classées en plusieurs immunotypes (IT L1 à L13), en fonction de leur réactivité avec une série d'anticorps qui reconnaissent des épitopes variés sur le LOS (Achtman et al, 1992, J. Infect. Dis. 165 : 53-68). Par la suite, le LOS de ces souches est répertorié de même en 13 immunotypes (IT L1 à L13). Les différences entre immunotypes proviennent de variations dans la composition et dans la conformation des chaînes oligosaccharidiques. Ceci transparaît dans le tableau ci-dessous, dérivé du tableau 2 de Braun et al, Vaccine (2004) 22 : 898, complété par des données obtenues ultérieurement et relatives aux immunotypes L9 (Choudhury et al, Carbohydr. Res. (2008) 343 : 2771) et L11 (Mistretta et al, (2008) Poster at the 16th International Pathogenic Neisseria Conference, Rotterdam) :

KDO
α2-4
β1-4 α1-5 α2-6
α chain R1 - Glc - Hep - KDO - Lipid A
α1-3
β chain R2 - Hep – R3
α1-2
GlcNAc
γ chain

| **IT** | **chaîne α (incluant le substituent R1)** | **R1** | **R2** |
|---|---|---|---|
| **L1** | NeuNAcα2-6Galα1-4Galβ1-4Glcβ1-4 | PEA-3 | - |
| **L2** | NeuNAcα2-3Galβ1-4GlcNAcβ1-4Glcβ1-4 | Glcα(1-3)** | PEA-6 ou PEA-7 |
| **L3** | NeuNAcα2-3Galβ1-4GlcNAcβ1-3Galβ1-4Glcβ1-4 | PEA-3 | - |
| **L4** | NeuNAcαL2-3Galβ1-4GlcNAcβ1-3Galβ1-4Glcβ1-4 | - | PEA-6 |
| **L5** | NeuNAcα2-3Galβ1-4GlcNAcβ1-3Galβ1-4Glcβ1-4Glcβ1-4 | Glcα(1-3) | - |
| **L6** | GIcNAcβ1-3Galβ1-4 Glcβ1-4 | - | PEA-6 ou PEA-7 |
| **L7** | Galβ1-4GlcNAcβ1-3Galβ1-4Glcβ1-4 | PEA-3 | - |
| **L8** | Galβ1-4 Glcβ1-4 | PEA-3 | - |
| **L9** | Galβ1-4GlcNAcβ1-3Galβ1-4 Glcβ1-4 | - | PEA-6 |
| **L10** | n.d. | n.d. | n.d. |
| **L11** | Glcβ1-4Glcβ1-4 | PEA-3 | PEA-6. |
| **L12** | n.d | n.d. | n.d. |
| **L13** | n.d | n.d. | n.d. |

| | | | |
|---|---|---|---|
| n.d. : non déterminé. ** Lorsque R2 est un résidu glucose, R2 est communément appelé chaîne β. | | | |

Entre autre, on peut noter que certains LOS peuvent être sialylés (présence d'acide N-acétyl neuraminique sur le résidu galactose (Gal) terminal de la chaîne α). Ainsi, les immunotypes L3 et L7 ne diffèrent que par la présence / absence respective de cette sialylation. Par ailleurs, la plupart des LOS sont substitués par un groupement O-acétyle sur le résidu glucosamine (α-GlcNAc ou chaîne γ) du core interne (Wakarchuk et al. (1998) Eur. J. Biochem. 254: 626 ; Gamian et al. (1992) J. Biol. Chem. 267: 922 ; Kogan et al (1997) Carbohydr. Res. 298 : 191 ; Di Fabio et al. (1990) Can. J. Chem. 68 : 1029 ; Michon et al. (1990) J. Biol. Chem. 275 : 9716 ; Choudhury et al. (supra) ; et Mistretta et al. (supra).

Le motif de carbohydrates Galβ1-4GlcNAcβ1-3Galβ1-4Glcβ1-4 ou motif lacto-N-neotetraose qui est présent dans la chaîne α de certains immunotypes du LPS de *N. meningitidis,* constitue un épitope qui peut potentiellement réagir de manière croisée avec les érythrocytes humains. Ainsi, en vue de fabriquer un vaccin destiné aux humains, il convient de choisir un LPS ne possédant pas ce motif. Il peut donc être particulièrement avantageux d'utiliser un LOS d'immunotype L8.

De manière alternative, on peut aussi envisager de partir par exemple d'une souche d'immunotype L2 ou L3 dans laquelle on a inactivé par mutation un gène intervenant dans la biosynthèse de la chaîne α, de manière à obtenir une structure LNnT incomplète. De telles mutations sont proposées dans la demande de brevet WO 04/014417. Il s'agit d'éteindre par mutation les gènes *lgt*B, *lgt*E (ou *lgt*H), *lgtA* ou *lgt*A et *Igt*C. Ainsi, il apparaît possible et intéressant d'utiliser un LPS provenant d'une souche de *N. meningitidis* d'immunotype L2 ou L3 *lgt*B⁻, *lgt*E⁻ (ou *lgt*H⁻), *lgt*A⁻ ou *lgt*A⁻ et *lgt*C⁻*.*

D'une manière générale on doit donc comprendre qu'un LPS utile aux fins de la présente invention peut posséder une structure telle que trouvée dans la nature ou bien ayant subi des modifications notamment à la suite de mutation.

Aux fins de la présente invention, le LPS peut être obtenu par des moyens conventionnels : en particulier, il peut être extrait à partir d'une culture bactérienne ; puis purifié selon des méthodes classiques. De nombreux procédés d'obtention sont décrits dans la littérature. A titre d'exemple, on cite *i.a*., Gu & Tsaï, 1993, Infect. Immun. 61 (5) : 1873 ; Wu et al, 1987, Anal. Biochem. 160 : 281 and US 6,531,131. Une préparation de LPS peut être quantifiée selon des procédures bien connues. Le dosage du KDO par chromatographie d'échange d'anions à haute performance (HPAEC-PAD) est une méthode qui convient tout particulièrement.

Quant au lipide A, il peut être obtenu *i.a.* par hydrolyse acide du LPS tel que par exemple décrit dans Gu & Tsai, Infect. Immun. (1993) 61 (5) : 1873.

### Le complexe

Le complexe décrit ou issu du procédé selon l'invention est un complexe de nature cationique (chargé positivement). De manière typique, ce peut être un liposome cationique.

Par « liposomes », on entend une entité synthétique, de préférence une vésicule synthétique, formée d'au moins une membrane (ou matrice) lipidique bi-couche refermée sur un compartiment aqueux. Aux fins de la présente invention, les liposomes peuvent être unilamellaires (une seule membrane bi-couche) ou plurilamellaires (plusieurs membranes disposées en oignon). Les lipides constituant la membrane bi-couche comportent une région non-polaire qui de manière typique est faite de chaîne(s) d'acides gras ou de cholestérol, et d'une région polaire, typiquement faite d'un groupement phosphate et/ou de sels d'ammonium tertiaires ou quaternaires. En fonction de sa composition, la région polaire peut, notamment à pH physiologique (pH ≈ 7), être porteuse d'une charge de surface nette (globale) soit négative (lipide anionique), soit positive (lipide cationique) ou ne pas porter de charge nette (lipide neutre).

Les complexes *i.a.,* les liposomes utiles aux fins de la présente invention, peuvent être n'importe quel type de complexes lipidiques de type cationique (porteurs de charges positives), *i.a.,* des liposomes cationiques. Au moins un des lipides entrant dans la composition de ces complexes *i.a.,* de ces liposomes, est un lipide cationique. Le ou les lipide(s) cationique(s) peuvent être accompagnés de lipides anioniques à condition que ces complexes *i.a*., ces liposomes, restent de nature cationique - c'est-à-dire que la charge globale des complexes *i.a*., des liposomes reste positive.

### Le lipide cationique

Aux fins de la présente invention, le lipide cationique peut être :
(i) les lipides incorporant une structure éthylphosphocholine tels les dérivés cationiques des phospholipides, en particulier les dérivés ester phosphoriques de la phosphatidylcholine, par exemple ceux décrits dans la demande de brevet WO 05/049080 et incluant notamment :
   - la 1,2-dimyristoyl-*sn*-glycéro-3-éthylphosphocholine,
   - la 1,2-dipalmitoyl-*sn*-glycéro-3-éthylphosphocholine,
   - la 1,2-palmitoyl-oleoyl-*sn*-glycéro-3-éthylphosphocholine,
   - la 1,2-distearoyl-*sn*-glycéro-3-éthylphosphocholine (DSPC),
   - la 1,2-dioleyl-*sn*-glycéro-3-éthylphosphocholine (DOEPC ou EDOPC ou ethyl-DOPC ou ethyl PC),
   - ainsi que leurs homologues structuraux ; et
(ii) les dérivés cationiques du cholestérol tels que le 3β-[N-(N',N'-dimethylaminoethane)-carbamoyl] cholestérol (DC-Chol) ou d'autres dérivés cationiques du cholestérol comme ceux décrits dans le brevet US 5 283 185 et notamment l'iodure de cholestéryl-3β-carboxamidoéthylènetriméthylammonium, la cholestéryl-3β-carboxyamidoéthylènamine, l'iodure de cholestéryl-3β-oxysuccinamido-éthylènetriméthylammonium et le 3β-[N-(polyéthylènimine)-carbamoyl]-cholestérol.

Par « homologues structuraux », on signifie les lipides qui possèdent la structure caractéristique du lipide de référence tout en s'en différenciant par des modifications secondaires, notamment au niveau de la région non-polaire, en particulier du nombre d'atomes de carbone et des doubles liaisons des chaînes d'acides gras.

Ces acides gras, que l'on trouve aussi dans les phospholipides neutre et anioniques, sont par exemple l'acide dodécanoique ou laurique (C12 :0), l'acide tétradécanoique ou myristique (C14:0), l'acide hexadécanoique ou palmitique (C16:0), l'acide cis-9-hexadecanoique ou palmitoleique (C16:1), l'acide octadécanoique ou stéarique (C18 :0), l'acide cis-9-octadécanoique ou oleique (C18 :1), l'acide cis,cis-9,12-octadécadiénoiquec ou linoléique (C18 :2), l'acide cis-cis-6,9-octadécadiénoique (C18 :2), l'acide all cis-9,12,15-octadécatriénoique ou α-linolénique (C18 :3), l'acide all cis-6,9,12-octadécatriénoique ou γ-linolénique (C18 :3), l'acide eicosanoique ou arachidique (C20 :0), l'acide cis-9-eicosénoique ou gadoleique (C20 :1), l'acide all cis-8,11,14-eicosatrienoique (C20:3), l'acide all cis-5,8,11,14-eicosatetraenoique ou arachidonique (C20 :4), l'acide all cis-5,8,11,14,17-eicosapentaneoique (C20 :5), l'acide docosanoique ou behenique (C22:0), l'acide all cis-7,10,13,16,19-docosapentaenoique (C22:5), l'acide all cis-4,7,10,13,16,19-docosahexaenoique (C22 :6) et l'acide tétracosanoique ou lignocérique (C24 :0) .

La structure caractéristique de l'EDOPC est :

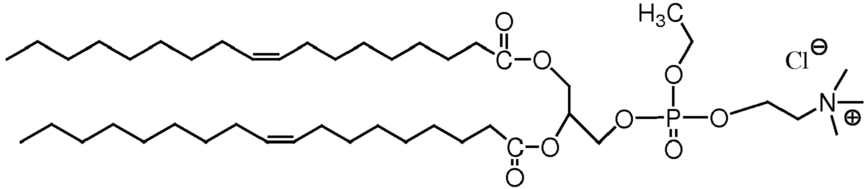

La structure caractéristique du DC-chol est :

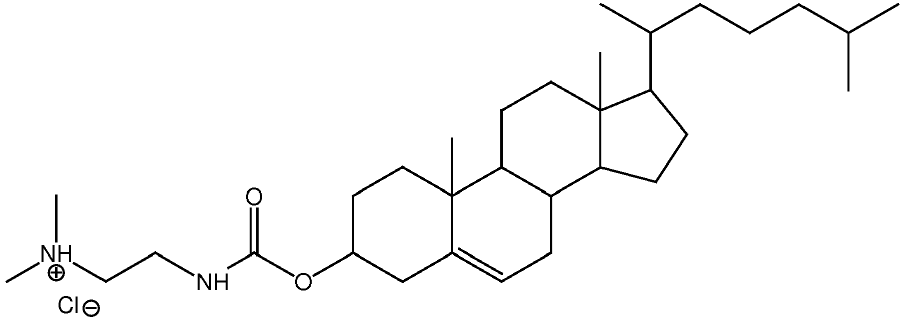

D'une manière générale, le lipide cationique peut être avantageusement utilisé en association avec un lipide neutre qui est souvent désigné sous le terme de co-lipide. Selon un mode de mélange avantageux, le rapport molaire lipide chargé (lipide cationique avec ou sans lipide anionique) : lipide neutre est compris entre 10 : 1 et 1 : 10, avantageusement entre 4 : 1 et 1 : 4, de préférence entre 3 : 1 à 1 : 3, bornes incluses.

En ce qui concerne les lipides neutres, on cite à titre d'exemple : (i) le cholestérol ; (ii) les phosphatidylcholines comme par exemple les 1,2-diacyl-*sn-*glycéro-3-phospho-cholines *e.g.,* la 1,2-dioleoyl-*sn*-glycéro-3-phosphocholine (DOPC), ainsi que les 1-acyl-2-acyl-*sn*-glycéro-3-phosphocholines dont les chaînes acyles sont différentes entres elles (chaînes acyles mixtes) ; et (iii) les phosphatidyléthanolamines comme par exemple les 1,2-diacyl-*sn*-glycéro-3-phosphoéthanolamines *e.g.,* la 1,2-dioleoyl-*sn-*glycéro-3-phosphoéthanolamine (DOPE), ainsi que les 1-acyl-2-acyl-sn-glycéro-3-phosphoéthanolamine portant des chaînes acyles mixtes.

Ainsi, selon un mode particulier, on utilise un mélange de lipide cationique et de lipide neutre. A titre d'exemple, on cite :
- un mélange de DC-chol et de DOPE, notamment dans un rapport molaire DC-chol : DOPE allant de 10 : 1 à 1 : 10, avantageusement de 4 : 1 à 1 : 4, de préférence d'environ 3 : 1 à 1 : 3;
- un mélange de EDOPC et de cholestérol, notamment dans un rapport molaire EDOPC : cholestérol allant de 10 : 1 à 1 : 10, avantageusement de 4 : 1 à 1 : 4, de préférence d'environ 3 : 1 à 1 : 3; et
- un mélange de EDOPC et de DOPE, notamment dans un rapport molaire EDOPC : DOPE allant de 10 : 1 à 1 : 10, avantageusement de 4 : 1 à 1 : 4 de préférence d'environ de 3 : 1 à 1 : 3.

Plusieurs techniques connues de l'homme de métier permettent d'obtenir des préparations de liposomes contenant du LPS (liposomes [LPS]). Ces différentes techniques peuvent être plus ou moins appropriées selon la nature et les propriétés du LPS, en particulier en fonction de sa solubilité en milieu aqueux ou organique. L'homme du métier est à même de sélectionner la ou les méthodes qui conviennent le mieux.

On peut par exemple incorporer le LPS dans des liposomes en préparant un film lipidique sec que l'on réhydrate ensuite avec une solution aqueuse de LPS comme décrit par Dijkstra et al, J. Immunol. Methods (1988) 114: 197-205. De manière alternative, si le LPS est soluble dans le solvant organique utilisé pour dissoudre les lipides, on peut directement préparer une solution organique contenant à la fois le LPS et les lipides, puis en séchant ce mélange lipidique sous forme d'un film sur les parois d'un récipient et enfin en réhydratant ce film lipidique sec par un tampon aqueux afin de former des liposomes contenant du LPS. De manière générale, l'étape de reconstitution en milieu aqueux conduit à la formation spontanée de vésicules multi-lamellaires dont la taille est ensuite homogénéisée par diminution progressive du nombre de lamelles par extrusion, par exemple à l'aide d'un extrudeur par passages de la suspension lipidique sous pression d'azote, à travers des membranes en polycarbonates de diamètres de pores de plus en plus réduits (0.8, 0.4, 0.2 µm).

On peut aussi incorporer le LPS dans des liposomes par la méthode dite de "deshydratation-réhydratation" dans laquelle des liposomes préformés sont mélangés avec du LPS en solution aqueuse, soniqués et lyophilisés avant d'être repris dans un tampon aqueux. Cette technique est par exemple utilisée par Petrov et al, Infect. Immun. (1992) 60 : 3897-3903.

On peut aussi incorporer le LPS dans des liposomes par la technique dite de "dilution de détergent" dans laquelle des micelles mixtes de LPS/lipides en détergent sont fortement diluées dans un tampon aqueux de manière à atteindre une concentration en détergent inférieure à la concentration micellaire critique du détergent. Il se forme alors des liposomes de LPS. Cette technique est par exemple utilisée par Argita et al, Vaccine (2005) 23 : 5091-5098. Cette méthode est équivalente à la méthode dite de "dialyse de détergent" décrite dans les exemples / données expérimentales qui font suite.

Selon un mode de préparation avantageux, on réalise dans un premier temps, un film lipidique sec avec tous les composés entrant dans la composition des complexes *i.a.* des liposomes. Puis on reconstitue le film lipidique en milieu aqueux, en présence de LPS, par exemple dans un rapport molaire lipides : LPS de 100 à 500, de manière avantageuse de 100 à 400 ; de préférence de 200 à 300 ; de manière tout particulièrement préférée, de 250 environ. D'une manière générale, on estime que ce même rapport molaire ne devrait pas varier de manière substantielle en final du procédé de préparation des liposomes LPS.

De manière générale, l'étape de reconstitution en milieu aqueux conduit à la formation spontanée de vésicules multi-lamellaires dont la taille est ensuite homogénéisée par diminution progressive du nombre de lamelles par extrusion, par exemple à l'aide d'un extrudeur par passages de la suspension lipidique sous pression d'azote, à travers des membranes en polycarbonates de diamètres de pores de plus en plus réduits (0.8, 0.4, 0.2 µm). On peut aussi remplacer le procédé d'extrusion par un autre procédé mettant en oeuvre un détergent (tensio-actif) qui disperse les lipides (voir Argita et al (supra)). Ce détergent est ensuite éliminé par dialyse ou par adsorption sur des microbilles de polystyrène poreuses particulièrement affines de détergent (BioBeads). Quand le tensio-actif se retire de la dispersion lipidique, les lipides se réorganisent en double couche.

A l'issue de la complexation *i.a.* de l'incorporation du LPS en liposomes, on peut communément obtenir un mélange constitué de complexes *i.a*., de liposomes *adhoc* et de LPS forme libre. Avantageusement, les liposomes sont alors purifiés afin de se débarrasser du LPS sous forme libre.

Compte tenu des propriétés du LPS et du lipide A, un complexe présentement décrit - peut avoir une utilisation variable : soit à titre d'adjuvant dans un vaccin quelconque comprenant un antigène vaccinal quelconque ; soit à titre d'antigène vaccinal dans un vaccin anti-bactérie à Gram-négatif ; soit à double titre d'adjuvant et d'antigène vaccinal.

### Vaccin / Usage à titre d'antigène vaccinal

Est également décrit une composition vaccinale (aussi dénommée vaccin) qui comprend un complexe LPS-lipide cationique obtenu selon un procédé selon l'invention ; le LPS étant présent en quantité suffisante pour que la réponse immune anti-LPS induite par le vaccin soit capable de protéger un individu contre une maladie générée par la bactérie à Gram-négatif.

Une composition vaccinale présentement décrite est notamment utile pour traiter ou prévenir une infection à bactérie à Gram-négatif non-entérique (telles que les bactéries des genres *Neisseriae, Bordetellae, Branhamellas, Haemophilus* et *Moraxellae) ;* ou des genres *Klebsiella, Pseudomonas, Burkolderia, Porphyromonas, Franciscella, Yersinia, Enterobacter, Salmonella, Shigella, Escherichia e.g, E. coli.*

Sous un aspect préféré, une composition vaccinale présentement décrite est notamment utile pour traiter ou prévenir une infection à *N. meningitidis,* telles que les méningites à *N. meningitidis,* les méningococcémies et les complications qui peuvent en dériver telles que le *purpura fulminans* et le choc septique ; ainsi que les arthrites et péricardites à *N. meningitidis.*

Elle peut être fabriquée de manière conventionnelle. En particulier, on associe une quantité de LPS efficace d'un point de vue thérapeutique ou prophylactique, à un support ou à un diluant.

Un vaccin présentement décrit peut en outre comprendre un adjuvant. Selon un mode avantageux l'adjuvant peut être un lipopolypeptide.

Ce dernier est typiquement un polypeptide sur lequel est (sont) greffé(s) par liaison covalente, un ou plusieurs lipide(s) incluant notamment les acides gras, les isoprénoïdes et le cholestérol, au niveau d'un ou plusieurs site(s) de lipidation.

Un adjuvant avantageux est un polypeptide acylé c'est-à-dire porteur d'au moins un, de préférence d'au moins deux ou trois groupements acyles. De manière avantageuse, le lipopolypeptide est bi ou tri acylé. Le ou les groupements acyles portés par le polypeptide peuvent être chacun de manière indépendante un groupement acyle en C8 à C22, de préférence en C 12 à C18, de manière tout particulièrement préférée en C14, C16 ou C18.

Ainsi, n'importe quel polypeptide acylé, de préférence au moins bi acylé, peut être utilisé à titre d'adjuvant. Il peut être d'origine naturelle ou synthétique. Un lipopolypeptide d'origine naturelle peut être celui d'un organisme procaryote ou eucaryote.

Dans la nature, les protéines sont modifiées par des lipides, de manière covalente, grâce à des mécanismes multiples tels que par exemple la N-myristoylation, la S-palmitoylation, la prénylation. La N-myristoylation consiste en l'addition covalente d'un acide gras, l'acide myristique, sur un résidu Glycine en position N-terminale, par une liaison amide. La S-palmitoylation consiste en l'addition covalente d'un acide gras, l'acide palmitique, sur un résidu Cysteine, par une liaison thio-ester. La prénylation consiste en l'addition d'un isoprénoïde, soit un groupement C15 farnesyl ou un C20 geranylgeranyl, sur un résidu Cystéine en position C-terminale, par une liaison thio-ester.

Ainsi, des lipopolypeptides avantageux peuvent être des polypeptides S-, N- et/ou O-acylés. A titre d'exemple, on cite des lipopolypeptides présentant une ou plusieurs des caractéristiques a) à c) suivantes :
a) la séquence d'acides aminés du polypeptide comprend au moins un résidu Cystéine qui est S-acylé par un groupement acyl R ;
b) l'acide aminé en position N-terminale, par exemple un résidu Glycine ou Cystéine, est N-acylé par un groupement acyl R' ;
c) l'acide aminé en position C-terminale est O-acylé par un groupement acyl R";
chacun des groupements R, R' et R" étant de manière indépendante, un groupement acyl comprenant de 8 à 22, de manière avantageuse de 10 à 20, de préférence de 12 à 18, de manière tout particulièrement préférée 14, 15, 16, 17 ou 18 atomes de carbone.

Selon un mode particulièrement avantageux, l'adjuvant lipopolypeptidique est une lipoprotéine procaryote, notamment une lipoprotéine bactérienne, par exemple d'une bactérie à Gram-négatif. Une grande partie des lipoprotéines bactériennes sont des protéines d'enveloppe, cette dernière étant constituée de deux membranes, externe et interne, délimitant dans l'entre-deux, un espace appelé périplasme.

D'une manière générale, les lipoprotéines de l'enveloppe bactérienne sont dans leur environnement naturel, des protéines ancrées à la face périplasmique de l'une des deux membranes par trois acides gras en position N-terminale. Deux, sous forme de diacylglyceryl, réduisent le groupement sulfhydryl (SH) du résidu Cystéine en position N-terminale (liaison thio-ether). Le troisième acyle le groupement amine (NH₂) de ce même résidu.

Selon un mode préféré, le lipopolypeptide en usage est une lipoprotéine d'une bactérie à Gram-négatif, entérique ou non-entérique, de préférence pathogène. Sous un aspect particulier, il peut s'agir d'une lipoprotéine de bactéries non-entériques des genres tels que les *Neisseriae, Bordetellae, Branhamellas, Haemophilus* et *Moraxellae.* Il peut s'agir aussi d'une lipoprotéine des genres *Klebsiella, Pseudomonas, Burkolderia, Franciscella, Yersinia, Enterobacter, Salmonella, Shigella et Escherichia, e.g. E. coli.*

Selon un mode tout particulièrement préféré, le LPS et le lipopolypeptide utilisé à titre d'adjuvant proviennent tous deux d'une bactérie à Gram-négatif, avantageusement de la même espèce bactérienne.

D'une manière générale, le lipopolypeptide, et plus particulièrement la partie « polypeptide » de ce dernier, comporte un ou des épitopes T-helper - c'est-à-dire des épitopes capables d'être reconnus par les cellules-T helper - et de les activer. Avantageusement, il s'agit d'épitopes T-helper caractéristiques de l'organisme auquel le vaccin à base de LPS est destiné (un mammifère, notamment un humain) - c'est-à-dire d'épitopes capables d'être reconnus par les cellules-T helper de l'organisme receveur et de les activer.

A titre indicatif, on cite les lipoprotéines suivantes : OspA (outer surface protein A) de *Borrelia Burgdorferi* ; la GNA1870 (protéine liant le facteur H) et la NMB1969 (NalP ou AspA) de *N. meningitidis* et la lipoprotéine D d'*Haemophilus* influenzae ou un fragment lipidé de ces lipoprotéines, notamment un fragment N-terminal.

Selon un mode préféré, un lipopolypeptide utile à titre d'adjuvant peut être la sous-unité B (TbpB) du récepteur de la transferrine humaine qui est une protéine de la membrane externe d'un certain nombre de bactéries à Gram négatif non-entériques telles que les *Neisseriae, Moraxellas, Branhamellas, Bordetellas* et *Haemophilus.* De manière alternative, il peut aussi s'agir d'un fragment N-terminal lipidé d'une TbpB. De manière tout particulièrement préférée, il s'agit de la TbpB de *Neisseria meningitidis* ou d'un fragment lipidé de cette dernière.

La composition vaccinale présentement décrite peut aussi comprendre un ou plusieurs antigène(s) vaccinal(aux) additionnel(s). De manière avantageuse, ils peuvent être choisis parmi les protéines de l'espèce bactérienne d'où provient le LPS. En lien avec cette possibilité, une composition vaccinale présentement décrite peut en outre contenir un adjuvant additionnel autre que le lipopolypeptide, acceptable d'un point de vue pharmaceutique, destiné à adjuver le ou les antigène(s) vaccinal(aux) additionnel(s) ; néanmoins cette option n'est pas la plus attrayante en raison de sa complexité.

Les quantités de LPS et/ou d'adjuvant par dose de vaccin qui sont suffisantes pour atteindre les fins pré-citées, efficaces d'un point de vue immunogène, prophylactique ou thérapeutique, dépendent de certains paramètres incluant l'individu traité (adulte, adolescent, enfant ou nourrisson), la voie d'administration et de sa fréquence.

Ainsi, la quantité de LPS par dose suffisante pour atteindre les fins pré-citées est notamment comprise entre 5 et 500 µg, avantageusement entre 10 et 200 µg, de préférence entre 20 et 100 µg, de manière tout à fait préférée entre 20 et 80 µg ou entre 20 et 60 µg, bornes incluses.

Lorsque l'on adjuve le LPS par un lipopolypeptide, la quantité de ce dernier par dose peut être comprise entre 5 et 500 µg, avantageusement entre 10 et 200 µg, de préférence entre 20 et 100 µg, de manière tout à fait préférée entre 20 et 80 µg ou entre 20 et 60 µg, bornes incluses.

Dans ce dernier cas, le rapport molaire LPS : lipopolypeptide peut être de 10⁻² à 10³, de manière avantageuse de 10⁻¹ à 10² ; de préférence de 1 à 50 ; de manière tout particulièrement préférée, de 15 à 30 ou de 20 environ.

Le terme « dose » employé ci-dessus doit être entendu comme désignant un volume de vaccin administré à un individu en une seule fois - c'est-à-dire à un temps T. Des doses conventionnelles sont de l'ordre du millilitre, par exemple 0.5, 1 ou 1.5 ml ; le choix définitif dépendant de certains paramètres et notamment de l'âge et du statut du receveur. Un individu peut recevoir une dose répartie en plusieurs sites d'injection le même jour. La dose peut être unique ou si nécessaire, l'individu peut aussi recevoir plusieurs doses à un certain délai d'intervalle - délai d'intervalle pouvant être déterminé par l'homme de l'art.

Une composition vaccinale présentement décrite peut être administrée par n'importe quelle voie conventionnelle en usage dans le domaine de l'art, *e.g.* dans le domaine des vaccins, notamment par voie entérale ou parentérale. L'administration peut avoir lieu en dose unique ou répétée avec un certain délai d'intervalle. La voie d'administration varie en fonction de divers paramètres par exemple de l'individu traité (condition, âge, etc.).

Enfin est également décrit :
- Une méthode pour induire chez un mammifère, par exemple un humain, une réponse immune à l'encontre d'une bactérie pathogène à Gram-négatif, selon laquelle on administre au mammifère une quantité efficace d'un point de vue immunogénique d'un vaccin présentement décrit pour induire une réponse immune, en particulier une réponse immune protectrice à l'encontre de la bactérie pathogène à Gram-négatif ; et
- Une méthode de prévention et/ou de traitement d'une infection induite par une bactérie pathogène à Gram-négatif selon laquelle on administre une quantité efficace d'un point de vue prophylactique ou thérapeutique d'un vaccin présentement décrit à un individu ayant besoin d'un tel traitement.

### Données Expérimentales

### 1. Préparation du LPS purifié

### Culture

Huit mL de congelât d'une souche de *N. meningitidis* serotype A connue pour exprimer de manière exclusive du LPS d'immunotype L8 (souche A1) servent à ensemencer 800 mL de milieu Mueller Hinton (Merck) supplémentés avec 4 mL d'une solution de glucose à 500 g/L et répartis en erlens. La culture est poursuivie sous agitation à 36 ± 1°C pendant environ 10 heures.

On ajoute à la pré-culture 400 mL d'une solution de glucose à 500 g/L et 800 mL d'une solution d'acides aminés. Cette préparation sert à ensemencer un fermenteur contenant du milieu Mueller-Hinton, à une DO₆₀₀ₙₘ proche de 0.05. La fermentation est poursuivie à 36°C, à pH 6.8, 100 rpm pO₂ 30 % sous un flux d'air initial de 0.75 L/min/L de culture.

Après environ 7 hrs (DO₆₀₀ₙₘ d'environ 3), on ajoute du milieu Mueller-Hinton à raison, de 440 g/hr. Quand la concentration en glucose est inférieure à 5 g/L la fermentation est arrêtée. La DO₆₀₀ₙₘ finale est couramment comprise entre 20 et 40. Les cellules sont récoltées par centrifugation et les culots congelés à -35°C.

### Purification

Les culots sont décongelés et suspendus avec 3 volumes de phénol 4.5 % (vol./vol.) en agitant vigoureusement pendant 4 hrs à environ 5°C. Le LPS est extrait par traitement au phénol.

La suspension bactérienne est chauffée à 65°C puis mélangée vol./vol. avec du phénol à 90 % en agitant vigoureusement pendant 50-70 min à 65°C. La suspension est ensuite refroidie à température ambiante puis centrifugée pendant 20 min à 11 000 g. La phase aqueuse est prélevée et conservée tandis que la phase phénolique et l'interphase sont récoltées pour être soumis à une deuxième extraction.

La phase phénolique et l'interphase sont chauffées à 65°C, puis mélangées à un volume d'eau équivalent à celui de la phase aqueuse précédemment prélevée en agitant vigoureusement pendant 50-70 min à 65°C. La suspension est ensuite refroidie à température ambiante puis centrifugée pendant 20 min à 11 000 g. La phase aqueuse est prélevée et conservée tandis que la phase phénolique et l'interphase sont récoltées pour être soumis à une troisième extraction identique à la seconde.

Les trois phases aqueuses sont dialysées séparément contre 40 L d'eau chacune. Puis les dialysats sont rassemblés ensemble. A 9 volumes de dialysat on ajoute un volume de Tris 20 mM, MgCl₂ 2mM. Le pH est ajusté à 8.0 ± 0.2 avec de la soude 4N.

Deux cent cinquante unités internationales de DNAse sont ajoutées par gramme de culot bactérien traité (poids humide). La préparation est agitée à 37°C pendant environ 1 hr. Le pH est ajusté à 6.8 ± 0.2. Puis la préparation est soumise à une fitration sur membrane de 0.22 µm et le filtrat purifié par passage sur une colonne de Sephacryl S-300 (5.0 x 90 cm ; Pharmacia™).

Les fractions contenant le LPS sont rassemblées ensemble et la concentration en MgCl₂ est élevée à 0.5 M en rajoutant de la poudre de MgCl₂,6H₂O sous agitation.

Tout en poursuivant l'agitation, on ajoute de l'alcool absolu déshydraté pour une concentration finale de 55 % (vol./vol.). L'agitation est poursuivie une nuit à 5 ± 2°C, puis on centrifuge à 5,000 g pendant 30 min à 5 ± 2°C. Les culots sont resuspendus avec au moins 100 mL de MgCl₂ 0.5 M puis soumis à une deuxième précipitation alcoolique identique à la précédente. Les culots sont resuspendus avec au moins 100 mL de MgCl₂ 0.5 M.

La suspension est soumise à une filtration sur gel comme précédemment décrit. Les fractions contenant le LPS sont rassemblées ensemble, stérilisées par filtration (0.8-0.22 µm) et conservées à 5 ± 2°C.

Ce procédé de purification permet d'obtenir environ 150 mg de LPS L8 par litre de culture.

### 2. Préparation des liposomes LPS (i.a., lipides : EDOPC et DOPE)

### 2.1. Production des liposomes [LPS L8] par dialyse de détergent

Les liposomes LPS L8 sont préparés par dialyse de détergent. En bref, les lipides (EDOPC : DOPE) sont mis sous forme de film lipidique et repris en tampon Tris 10 mM, puis dispersés en octyl β-D-glucopyranoside (OG ; Sigma-Aldrich ref O8001) 100 mM et filtrés stérilement. Le LPS L8 en OG 100 mM est ajouté stérilement. Le mélange lipides/LPS/OG est ensuite dialysé en tampon Tris 10 mM pour éliminer l'OG et former les liposomes.

### Protocole

On réalise une préparation lipidique en chloroforme des lipides que l'on va utiliser pour la fabrication des liposomes. Un film sec est obtenu par évaporation complète du chloroforme.

Par exemple, un film sec de 1,2 dioleyl-*sn*-glycero-3-ethylphosphocholine (EDOPC ou ethyl-DOPC) et de 1,2-dioleoyl-*sn*-glycero-3-phosphoethanolamine (DOPE) dans un rapport molaire EDOPC : DOPE de 3 pour 2 est obtenu en mélangeant 12.633 mL d'une solution de EDOPC (Avanti Polar Lipids ref 890704) à 20 mg/ml en chloroforme et 7.367 mL d'une solution de DOPE (Avanti Polar Lipids ref 850725) à 20 mg/ml en chloroforme et en évaporant le chloroforme jusqu'à disparition complète.

Le film sec est repris par 30 mL de Tris 10 mM pH 7.0 pour obtenir une suspension contenant 13.333 mg de lipides /ml (8.42 mg/ml d'EDOPC et 4.91 mg/ml de DOPE). La suspension est agitée 1 hr à température ambiante puis soniquée 5 min dans un bain. Puis on ajoute toujours sous agitation, 3.333 ml d'une solution stérile d'OG 1 M en Tris 10 mM pH 7.0 pour obtenir une suspension limpide de lipides à 12 mg/ml, OG 100 mM, Tris 10 mM. On poursuit l'agitation 1 hr à température ambiante sur table d'agitation. Puis on filtre stérilement sur Millex HV 0.45 µm.

On mélange dans des conditions stériles les préparations suivantes :
2.005 mL de Tris 10 mM pH 7.0 ; 0.223 mL d'OG 100 mM en Tris 10 mM ; 31.373 mL de la suspension EDOPC : DOPE de ratio molaire 3 : 2 à 12 mg/ml en OG 100 mM Tris 10 mM ; et 6.4 mL d'une suspension stérile de LPS L8 à 1 mg/ml en OG 100 mM Tris 10 mM ; pour obtenir ainsi 40 mL d'une composition ayant un rapport molaire lipides : LPS de 250 (0.160 mg/mL de LPS L8, 9.412 mg/mL et 100 mM d'OG).

Après une heure d'agitation à température ambiante, la suspension est transférée stérilement dans 4 cassettes de dialyse stériles de 10 ml. Chaque cassette est dialysée 3 fois (24 hrs - 24 hrs - 72 hrs) contre 200 volumes de Tris 10 mM/L pH 7.0 soit 2 L.

On récupère les liposomes dans des conditions stériles. L'augmentation de volume après dialyse est d'environ de 30 %.

A cette préparation on ajoute du merthiolate et du NaCl pour obtenir une préparation de liposomes en Tris 10 mM, NaCl 150 mM, pH 7.0, Merthiolate 0.001 %.

En final, la préparation est obtenue aux concentrations suivantes:
- LPS environ 110 µg/ml
- Lipides environ 7 mg/ml dont EDOPC environ 4.5 mg/ml et DOPE environ 2.5 mg/ml
- Tris 10 mM/L
- NaCl 150 mM/L
- Merthiolate 0.001 %

Les liposomes LPS sont conservés à +5°C.

### 2.2. Production des liposomes [LPS L8] par extrusion

On réalise des liposomes [LPS L8] avec du DC-chol ou de l'EDOPC dans un rapport molaire lipide/LPS de 250.

Pour ce faire, on dissout 129 µg de LPS et 5.2 mg de DC-chol ou 10.4 mg d'EDOPC dans 10 mL d'un mélange chloroforme / méthanol 4: 1. Un film sec est réalisé par évaporation de solvant et séchage complémentaire sous vide poussé. Le film est repris par de l'eau ultra-filtrée à 50°C au vortex. La préparation est passée au bain à ultrasons ; puis soumise à extrusion par filtration sur membrane (seuil de rétention : 0.4 µm) une fois ; suivie de six filtrations consécutives sur membrane (seuil de rétention : 0.2 µm). On stérilise par filtration.

### 3. Evaluation de la détoxification du LPS en liposomes

Trois tests principaux sont utilisés : (i) le test LAL (Limulus Amebocyte Lysate) ; (ii) un test *in vitro* de relargage des cytokines IL6 et TNFα ; et (iii) le test pyrogène chez le lapin.

### Test LAL

Le test LAL est un test colorimétrique très sensible permettant de détecter et de quantifier les endotoxines des bactéries Gram-négatif. Ce test est mis en oeuvre selon les règles de la Pharmacopée Européenne (Edition 5.0., paragraphe 2.6.14.) en utilisant le kit QCL-1000 ref ; 50-647 U de Cambrex-BioWhittaker™ (zone linéaire du test : 0.1 à 1 UI/mL) avec pour contrôle positif, l'endotoxine d'*E. coli,* 4.10³ EU/mL (Sigma™).

On procède aux dilutions (i) des échantillons à tester, (ii) du standard et (iii) du contrôle positif dans les gammes respectives de 1/10 à 1/10⁵ ; 0.5 à 0.031 EU/mL ; et 1/10⁴ à 1.8 10⁴.

Cinquante µL des dilutions des échantillons, du standard et du contrôle positif sont répartis dans les puits d'une plaque ELISA de 96. 50 µL de lysat sont ajoutés dans chacun des puits, puis 100 µL de p-nitroaniline. L'incubation est poursuivie 6 min à 37°C. La réaction est arrêtée en ajoutant 100 µL d'acide acétique glacé à 25 % (dans de l'eau). La plaque est lue par spectrométrie à 405 nm.

Calcul de la concentration d'endotoxines : On soustrait à toutes les densités optiques (DO) la valeur moyenne de DO des échantillons « blanc ». On établit la droite de régression linéaire de la gamme standard (elle doit être linéaire de 0.031 EU/mL à 0.5 EU/mL) pour calculer la concentration d'endotoxine (EU/mL) de chaque échantillon testé à partir des DO obtenues. On multiplie ensuite ces valeurs par l'inverse des dilutions correspondantes et on calcule la moyenne arithmétique.

Le taux de détoxification est déterminé comme étant la valeur de LAL mesurée sur le LPS non formulé divisé par la valeur du LAL mesurée sur le produit formulé en liposomes à concentration en LPS équivalente.

### Test in vitro de relargage des cytokines

On dilue au cinquième, dans du milieu AIM-V (Invitrogen™), du sang humain collecté dans de l'héparine sodique (25,000 U/5mL ; Sanofi aventis). Cette préparation est répartie dans des tubes Micronics™ à raison de 400 µL par puits. On ajoute 100 µL des substances à tester. Les tubes sont incubés 24 hrs à 37°C dans une atmosphère humide chargée à 5 % de CO₂.

Les tubes sont centrifugés 10 min à 500 g. On prélève sur chaque tube au moins 200 µL du surnageant plasmatique que l'on garde congelé à -80°C jusqu'à ce que le titrage soit effectué.

Le titrage des cytokines est effectué en ELISA à l'aide des kits OptEIA IL6, IL8 et TNFα humaines de chez Pharmingen™, chacun des kits comprenant un anticorps de capture (anticorps de souris anti-cytokine humaine), un anticorps de détection (anticorps de souris anti-cytokine humaine biotinylé), du conjugué avidine-peroxydase et les standards.

Les anticorps de capture sont dilués au 250^{ième} dans un tampon carbonate 0.1 M pH 9.5 (Sigma™). Pour chaque test, 100 µL de la dilution au 250^{ième} sont distribués dans chaque puits d'une plaque ELISA de 96 à fond plat (Maxisorp NUNC 96™). Les plaques sont incubées une nuit à 4 °C.

Les plaques sont lavées dans du PBS 0.05 % Tween 20. On ajoute 200 µL de PBS 0.05 % sérumalbumine bovine par puits. L'incubation est poursuivie 1 hr à température ambiante. Les plaques sont lavées dans du PBS 0.05 % Tween 20.

On prépare des dilutions des cytokines recombinantes en milieu AIM-V dans les gammes suivantes : 1,200 pg/mL - 18.75 pg/mL (IL6) ; 800 pg/mL - 12.5 pg/mL (IL8); et 1,000 pg/mL - 15.87 pg/mL (TNFα). 100 µL de chaque dilution sont distribués dans les puits pour établir la courbe standard.

Les plasmas récoltés à partir du sang stimulé avec du LPS pur sont dilués au 25^{ième} et 125^{ième} Ceux récoltés à partir du sang en contact avec des liposomes LPS sont dilués au 5^{ième} et au 25^{ième}. 100 µL de chaque dilution sont distribués par puits. L'incubation est poursuivie 2 hrs à température ambiante.

Les plaques sont lavées dans du PBS 0.05 % Tween 20. L'anticorps de détection et l'enzyme sont tous deux dilués au 250^{ième} dans du PBS contenant 10 % de sérum de veau foetal. On ajoute 100 µl de chaque dilution par puits. L'incubation est poursuivie une heure à température ambiante.

Les plaques sont lavées dans du PBS 0.05 % Tween 20. On distribue 100µl de substrat par puits (solutions A et B de tétraméthylbenzidine mélangées vol. à vol). L'incubation est poursuivie 10 à 30 min à température ambiante.

On arrête la réaction en ajoutant 100 µL d'acide phosphorique 1 M par puits. Les plaques sont lues à 450 nm.

Les courbes standard de concentration de cytokines en fonction de la densité optique sont obtenues à partir d'une gamme de dilution de cytokines recombinantes, et les résultats bruts correspondent aux concentrations des échantillons obtenues à partir de ces courbes standards.

Le taux de détoxification est déterminé comme étant le rapport de la concentration de LPS formulé en liposomes induisant 50 % du maximum de relargage (ED50 exprimé en pg/mL) divisé par la concentration de LPS non formulé induisant 50 % du maximum de relargage de cytokine. Plus le taux est élevé, plus la détoxification est poussée. Comme le taux de détoxification est systématiquement mesuré en utilisant du sang de plusieurs donneurs indépendants, les résultats sont formés d'une moyenne.

### Test pyrogène chez le lapin

Le lapin est considéré comme étant l'animal ayant une sensibilité aux effets pyrogènes du LPS équivalente à celle de l'homme. Le test pyrogène consiste à mesurer l'élévation de température induite par une injection intraveineuse d'une solution stérile des substances à examiner. Le test, la lecture et les calculs sont mis en oeuvre selon les règles édictées par la pharmacopée européenne (Edition 6.0, paragraphe 2.6.8.). Un effet pyrogène est déclaré lorsque l'on observe une élévation de température de plus de 1.15°C.

### 4. Etude d'immunogénicité chez la souris

### Immunisation des souris

Des souris femelles CD1 (Charles River Lab.) âgées de 7 semaines, réparties en différents groupes, reçoivent par voie sous-cutanée un aliquote de 200 µl des préparations à tester ajustées en Tris 10mM NaCl 150mM pH 7.0, à 50 µg/mL en LPS.

Du sang est prélevé pour analyse avant chacune des deux injections. Les souris sont sacrifiées à J35.

### Dosage des anticorps anti-LPS par ELISA (IgG, IgM)

Ce dosage est robotisé (robot Staccato, Caliper) selon le protocole suivant :

Les puits de plaques Dynex™ de 96 puits sont imprégnés avec 1 µg de LPS L8 en tampon PB S (phosphate buffer saline) 1X, pH 7.1 ° MgCl₂ 10mM, incubées 2 heures à 37°C puis une nuit à 4°C. On bloque les plaques en ajoutant dans les puits 150 µl de PBS contenant 0.05 % de Tween 20 et 1 % (poids/vol.) de lait écrémé en poudre (PBS-Tween-lait). Les plaques sont incubées 1 hr à 37°C.

Des dilutions en série de deux en deux des échantillons à tester sont réalisées en PBS - Tween 0.05 % - lait 1 %. Les plaques sont incubées 90 min à 37°C puis lavées 3 fois avec du PBS + Tween 20 à 0.05 %.

Un conjugué peroxydase - anti-IgG ou anti-IgM de souris ou de lapins en PBS-Tween-lait est ajouté dans les puits et les plaques sont incubées 90 min à 37°C. Les plaques sont lavées trois fois. On distribue 100 µl par puits d'une solution de TMB (3,3',5,5'-tétraméthylbenzidine, substrat de la peroxidase) prête à l'emploi. Les plaques sont incubées dans l'obscurité pendant 20 min à température ambiante. La réaction est stoppée en ajoutant 100 µl de HCl 1M par puits.

La densité optique est mesurée à 450-650 nm avec un lecteur automatique (Multiskan Ascent). En l'absence de standard, les titres anticorps sont déterminés comme étant la dilution réciproque donnant une densité optique de 1.0 sur une courbe Tendance (logiciel CodUnit). Le seuil de détection des anticorps est de 1.3 log₁₀ unité ELISA. Pour chaque titre inférieur à ce seuil, une valeur arbitraire de 1.3 log₁₀ est attribuée.

### 5. Quantification du LPS et des lipides en liposomes

### 5.1. Dosage des lipides par HPLC-UV

### Préparation de la gamme d'étalonnage et des échantillons à analyser

On prépare une solution-mère contenant 1 mg/mL en chloroforme de chacun des lipides EDOPC, DOPC, DOPE, DC-chol et cholestérol que l'on dilue ensuite au 10^{ième} en ajoutant un mélange acétonitrile/eau (90/10). Cette solution mère sert à la préparation de la gamme d'étalonnage de 2 à 50 µl/ml par dilution dans le mélange acétonitrile/eau.

Les échantillons à analyser sont dilués en acétonitrile/eau pour parvenir à une concentration finale théorique d'environ 10 µg/ml.

### Conditions analytiques

On utilise une colonne Zorbax C18 Extend, 3 ;5 µm, 3 x 150 mm, 80Å, (Agilent référence 763954-302) et pour phase mobile un mélange acétonitrile/eau/acide trifluoroacétique (TFA) dans les proportions de volume 850/150/1. La colonne est conditionnée au préalable selon le processus suivant :
- débit à 0,25 ml/min pendant 20 minutes (P = 21 bars)
- débit à 0.5 ml/min pendant 20 minutes (P = 42 bars)
- débit à 0.75 ml/min pendant 20 minutes (P = 60 bars)
- débit à 1 ml/min pendant 20 minutes (P = 80 bars)

Les mesures sont effectuées à 60°C, en injectant 10 µL de la préparation sous un débit de phase mobile de 1 ml/min. Les analytes sont détectés à DO de 200 nm.

Temps de rétention moyen du DC-Chol : 1,6 minute

Temps de rétention moyen de l'EthylDOPC : 7,7 minutes

Temps de rétention moyen de la DOPC : 9,9 minutes

Temps de rétention moyen de la DOPE : 11,5 minutes

Temps de rétention moyen du Chol : 13,4 minutes

### 5.2. Dosage du LPS par HPAEC-PAD

Le principe du dosage consiste à soumettre le LPS à une hydrolyse acide qui libère une molécule de KDO par molécule de LPS ; puis à séparer ce KDO libre du reste et à le quantifier par chromatographie haute performance d'échange d'ions couplée à une détection ampérométrique pulsée (HPAEC-PAD).

### Préparation de la gamme étalon et des échantillons à doser

On prépare sous un volume final de 400 µL, un blanc et gamme étalon de KDO comprise entre 42.5 et 1700 ng/mL ; ce qui correspond à une gamme étalon en LPS comprise en 613 et 24507 ng de LPS/mL. Le blanc et chacun des échantillons de la gamme contient en outre une quantité de lipides et/ou de détergent sensiblement équivalente à celle présente dans les échantillons à doser; soit *e.g.,* 0.7 mg/ml d'un mélange d'EDOPC et de DOPE dans un rapport molaire de 3:2 ainsi que de l'octyl glucoside à 0.2 mM.

Les échantillons à doser sont préparés sous un volume final de 400 µL par dilution *e.g*. au 10^{ième} d'une préparation de liposomes à une concentration théorique en LPS de départ à 100 µg/mL.

### Hydrolyse acide

On distribue 100 µL d'une solution d'hydrolyse contenant de l'acide acétique 5 % et de l'acide glucuronique à 20 µg/mL (composé utilisé comme standard interne) préparée de manière extemporanée, dans les échantillons de la gamme étalon + blanc et dans les échantillons à doser. L'hydrolyse est poursuivie 1 hr à 100°C puis arrêtée par centrifugation à 5°c pendant 5 min.

### Extraction des lipides et du détergent

500 µl d'eau purifiée est additionnée à la solution hydrolysée précédente puis on ajoute 2 mL d'un mélange chloroforme / méthanol (2/1) et on mélange au vortex pendant 30 sec. On centrifuge à 4500 rpm/ min pendant 10 min. Les phases aqueuses sont prélevées, séchées à 45°C pendant 2 hrs sous un débit d'azote à 0.5 bar et reprises avec 400 µl d'eau.

### Dosage HPAEC-PAD

Cette technique est mise en oeuvre sur une chaîne HPAEC (Dionex™) en utilisant le logiciel de pilotage Chromeleon Dionex™ pour l'acquisition et le retraitement des données. La colonne de chromatographie (Carbopac PA1 4 x 250 mn Dionex™ référence 035391) est soumise à une température de 30°C. La colonne est équilibrée avec une solution d'élution (NaOH 75 mM, AcONa 90 mM) et conditionnée au préalable selon le schéma suivant :
- débit à 0,20 ml/min pendant 20 minutes (P = 270 psi)
- débit à 0.4 ml/min pendant 20 minutes (P = 540 psi)
- débit à 0.6 ml/min pendant 20 minutes (P = 800 psi)
- débit à 0.8 ml/min pendant 20 minutes (P = 1055 psi)
- débit à 1 ml/min pendant 20 minutes (P = 1300 psi)
100 µL d'un échantillon sont injectés sur la colonne sous un débit d'élution de 1 mL/min pendant 22 min.

La quantité de KDO présente dans l'échantillon est déterminée par l'intégration du pic KDO du chromatogramme. Puisque une mole de KDO est libérée par mole de LPS, il est possible de déterminer la concentration de LPS présente dans la préparation initiale.

### 6. Résultats

### 6.1. Supériorité des liposomes cationiques en matière de détoxification du LPS

Trois sortes de liposomes contenant du LPS ont été fabriqués selon la méthode par extrusion : (i) des liposomes contenant un seul lipide, celui-ci étant de type neutre (DOPC) ; (ii) des liposomes contenant un seul lipide, celui-ci étant de type cationique (EDOPC ou DC-chol) ; et (iii) des liposomes contenant un lipide cationique et un lipide neutre). La description de ces liposomes est fournie dans le tableau ci-après ainsi que les résultats du test LAL et du test pyrogène chez le lapin. Le LPS incorporé en liposomes neutres induit un effet pyrogène chez le lapin à des doses en LPS administrées qui ne produisent pas cet effet lorsque le LPS est incorporé dans des liposomes cationiques.

| Lipide 1 : Lipide 2 | Quantité de produit (LPS, lipide 1, lipide 2) utilisé pour fabriquer les liposomes | | | | LAL EU (moyenne de 3 tests) / µg de LPS | Test pyrogène (lapin) |
|---|---|---|---|---|---|---|
| | LPS (µg/ml) | Lipide 1 (µg/mL) | Lipide 2 (µg/mL) | Rapport molaire lipides : LPS | | |
| DC-chol | 9.92 | 400 | | 250 | 5080 (facteur de détoxification de 16) | Non-pyrogène à 10, 25 et 50 ng de LPS /ml |
| EDOPC : DOPE | 9.92 | 381 | 223 | | 824 (facteur de détoxification de 35) | |
| EDOPC | 9.92 | 635 | | | 12791 (facteur de détoxification de 2) | |
| DC-chol : DOPE | 9.92 | 201 | 222 | | 5335 | |
| DOPC (neutre) | 9.92 | 585 | | | | Pyrogène à 25 et 50 ng/ml |
| LPS non traité | 10 | | | | 28656 | |

### 6.2. Etude de la détoxification du LPS en fonction du rapport molaire lipides : LPS, de la composition et/ou du procédé de fabrication des liposomes

Différentes sortes de liposomes contenant du LPS ont été fabriqués soit par extrusion, soit par dialyse de détergent. Leur composition est décrite dans le tableau ci-après. La taille des liposomes fabriqués est analysée par diffusion quasi-élastique de la lumière sur un Malvern Zetasizer nano-S. Dans tous les cas cette taille est largement inférieure à 200 nm.

| | Lipides (mol/mol) | Technique | Valeurs théoriques (avant process) | | Concentrations réelles (après process) | | | |
|---|---|---|---|---|---|---|---|---|
| | | | Ratio molaire Lipide/LPS | LPS (µg/mL) | LPS (µg/mL) | EDOPC (µg/mL) | DOPE (µg/mL) | DC-chol ou Chol (µg/mL) |
| A | EDOPC : DOPE | Dialyse OG | | 100 | 81 | 215 | 90 | |
| B | | | 100 | | 78 | 1334 | 570 | |
| C | | | 175 | | 82 | 1796 | 980 | |
| D | (3 : 2) | | 250 | | 85 | 2678 | 1580 | |
| E | | | 325 | | 78 | 3365 | 2280 | |
| F | | | 400 | | 80 | 4148 | 2890 | |
| Fbis | | | 400 | | 86 | 3650 | 2680 | |
| G | EDOPC : chol. | Dialyse OG | 250 | | 64 | 2960 | | 530 |
| | (7 : 3) | | | | | | | |
| H | DC-chol | Extrusion | 250 | | 34 | | | 3080 |
| I | EDOPC | Extrusion | 250 | | 63 | 5151 | | |
| J | EDOPC : DOPE | Dialyse OG | - | 0 | | 3695 | 241 | |
| | (3 : 2) | | | | | | | |

La détoxification du LPS est mesurée à T0 et T + 3 mois par les méthodes LAL et dosage de cytokines. Les résultats sont présentés dans le tableau ci-après :

| | Lipides | Technique | Ratio molaire Lipide/LPS | Detoxification | | | |
|---|---|---|---|---|---|---|---|
| | | | | Relargage de l'IL6 | | LAL | |
| | (mol/mol) | | | T = 0 | T = +3 mois | T = 0 | T = +3 mois |
| A | EDOPC : DOPE | Dialyse OG | 20 | 2198 | 3252 | 4 | 43 |
| B | | | 100 | 751 | 2796 | 11 | 148 |
| C | | | 175 | 1229 | 6430 | 33 | 236 |
| D | (3:2) | | 250 | 1089 | 1574 | 163 | 270 |
| E | | | 325 | 860 | 1170 | 321 | 187 |
| F | | | 400 | 648 | 754 | 360 | 310 |
| G | EDOPC : chol. | Dialyse OG | 250 | 279 | 2359 | 166 | 157 |
| | (7 : 3) | | | | | | |
| H | DC-chol | Extrusion | 250 | 144 | 258 | 6 | 4 |
| I | EDOPC | Extrusion | 250 | 23 | 271 | 38 | 154 |
| J | EDOPC : DOPE | Dialyse OG | - | Pas de secretion d'IL6 | Pas de secretion d'IL6 | Nd | Nd |
| | (3 : 2) | | | | | | |
| LPS purifié | | | | 1 | 1 | 1 | 1 |
| Endotoxoid | | | | 49 | 65 | 2178 | Nd |

| | | | | | | | |
|---|---|---|---|---|---|---|---|
| Nd : Non déterminé. | | | | | | | |

Le taux de détoxification apprécié par relargage de l'IL6 est déterminé comme étant le rapport de la concentration de LPS formulé en liposomes induisant 50 % du maximum de relargage (ED50 exprimé en pg/mL) / concentration de LPS non-formulé induisant 50 % du maximum de relargage.

Dans le test LAL, le taux de détoxification est déterminé comme étant la valeur de LAL mesurée sur le LPS non formulé divisé la valeur du LAL mesurée sur le produit formulé en liposomes à concentration en LPS équivalente.

Même si on peut noter que les taux de détoxification semblent suivre la concentration en LPS dans les liposomes, les deux tests utilisés donnant d'ailleurs des tendances inverses, il n'est pas possible de conclure à des différences notables d'une concentration à une autre. La concentration en LPS dans les liposomes ne devrait pas avoir d'incidence sur sa détoxification. Par contre, l'addition d'un co-lipide (Cholestérol ou DOPE) au lipide de base (EDOPC) semble favoriser cette dernière.

La détoxification du LPS en liposomes est aussi comparée à celle obtenue avec l'endotoxoïde obtenu en complexant du LPS purifié avec le peptide SAEP2-L2 (dimérique, anti-parallèle) selon l'information donnée dans WO 06/108586. La détoxification est moindre mais tout à fait acceptable car le peptide SAEP2-L2 détoxifie le LPS au-delà de ce qui est requis.

### 6.3. Etude de l'immunogenicité du LPS en fonction du rapport molaire lipides : LPS, de la composition et/ou du procédé de fabrication des liposomes

Des souris groupées par 10 ont été immunisées à J0 et J21 avec 10 µg de LPS par injection d'un aliquote d'une préparation A-I de LPS en liposomes à 50 µg/ml en tampon Tris 10 mM, NaCl 150 mM, pH 7.0. Des contrôles négatifs et positifs sont ajoutés au test. Les contrôles positifs sont au nombre de deux : Le LPS purifié non-détoxifié du même lot (10 µg par injection) que celui ayant servi à fabriquer les liposomes LPS ainsi que 10 µg de LPS de ce lot sous forme d'endotoxoide - endotoxoide fabriqué selon WO 06/108586.

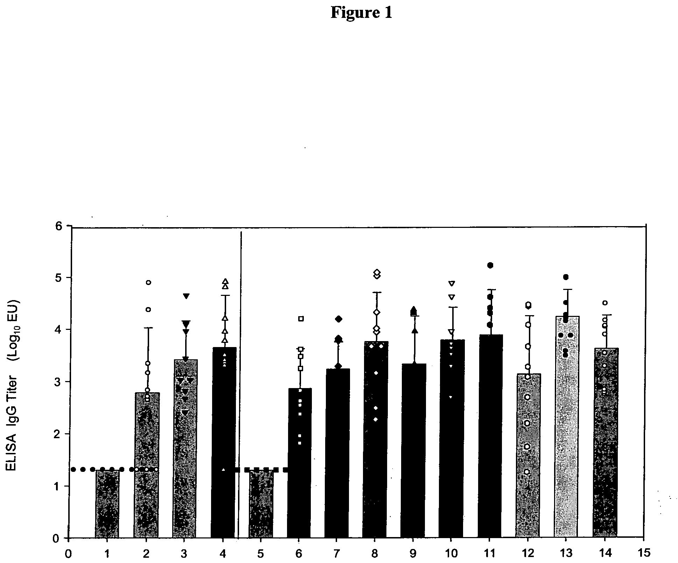

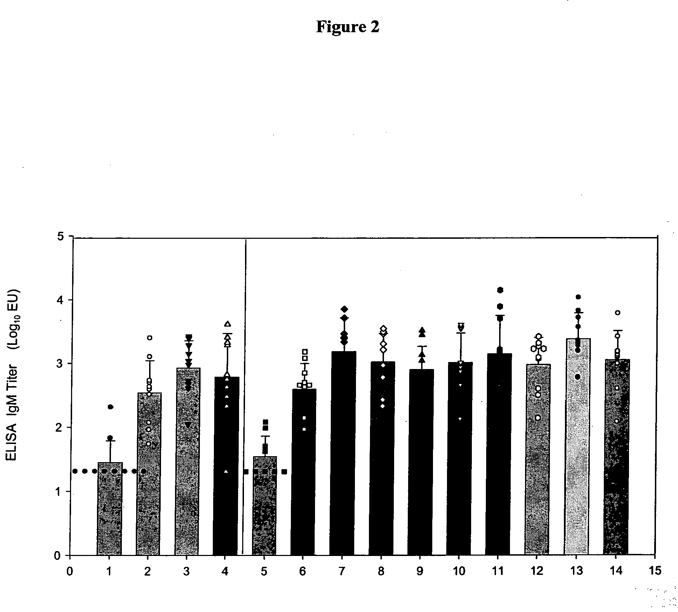

Les quantités d'IgG et d'IgM anti-LPS induites ont été évaluées par ELISA 35 jours après la première injection. Les résultats sont présentés dans les figures 1 (IgG) et 2 (IgM). Dans chacune de ces figures les numéros 6 à 14 et 5 renvoient respectivement aux échantillons A à J décrits dans les tableaux ci-avant. L'échantillon 1 est un échantillon témoin constitué uniquement d'une solution tampon. L'échantillon 3 contient du LPS L8 non-détoxifié. Les échantillons 2 et 4 sont les échantillons de référence contenant de l'endotoxoïde (LPS L8 détoxifié par complexation avec le peptide SAEP2 L2, analogue de la polymixine B)

On constate que quelque soit le mode de formulation du LPS, le caractère antigénique du LPS détoxifié présente une grande homogénéité. Sa capacité à induire des anticorps est tout à fait comparable à celles du LPS non détoxifié et de l'endotoxoide.

## Revendications

1. Un procédé de détoxification du lipopolysaccharide (LPS) ou du lipide A d'une bactérie à Gram-négatif selon lequel on mélange le LPS ou le lipide A avec un lipide cationique pour former un complexe dans lequel le LPS est associé au lipide cationique,
le lipide cationique étant sous forme de liposomes cationiques, et le LPS ou le lipide A étant incorporé auxdits liposomes, et
le lipide cationique étant sélectionné dans le groupe constitué par
(i) les lipides incorporant une structure éthylphosphocholine ; et
(ii) les dérivés cationiques du cholestérol.

2. Un procédé selon la revendication 1, dans lequel le LPS est le lipooligosaccharide (LOS) de *Neisseria meningitidis.*

3. Un procédé selon la revendication 2, dans lequel le lipide cationique est la 1,2-dioleyl-*sn-*glycéro-3-éthylphosphocholine (EDOPC) ou le 3β-[N-(N',N'-dimethylaminoethane)-carbamoyl] cholestérol (DC-Chol).

4. Un procédé selon l'une des revendications 1 à 3, selon lequel on mélange le LPS ou le lipide A avec le lipide cationique et en outre un lipide neutre (co-lipide) pour former un complexe dans lequel le LPS ou le lipide A est associé au lipide cationique et au lipide neutre.

5. Un procédé selon la revendication 4, dans lequel le lipide neutre est sélectionné dans le groupe constitué par (i) le cholestérol ; (ii) les phosphatidylcholines ; et (iii) les phosphatidyléthanolamines.

6. Un procédé selon la revendication 5, dans lequel le lipide neutre est la 1,2- dioleoyl-*sn-*glycéro-3-phosphoéthanolamine (DOPE).

## Patentansprüche

1. Verfahren zum Entgiften von Lipopolysaccharid (LPS) oder von Lipid A eines gramnegativen Bakteriums, bei dem man das LPS oder das Lipid A mit einem kationischen Lipid mischt, um einen Komplex zu bilden, in dem das LPS mit dem kationischen Lipid assoziiert ist,
wobei das kationische Lipid in Form von kationischen Liposomen vorliegt, und das LPS oder das Lipid A in die Liposomen eingebaut ist, und
wobei das kationische Lipid aus der Gruppe bestehend aus den Folgenden ausgewählt ist:
(i) Lipiden, die eine Ethylphosphocholinstruktur beinhalten; und
(ii) kationischen Derivaten von Cholesterin.

2. Verfahren nach Anspruch 1, wobei es sich bei dem LPS um das Lipooligosaccharid (LOS) von *Neisseria meningitidis* handelt.

3. Verfahren nach Anspruch 2, wobei es sich bei dem kationischen Lipid um 1,2-Dioleyl-sn-glycero-3-ethylphosphocholin (EDOPC) oder um 3β-[N-(N',N'-Dimethylaminoethan)carbamoyl]cholesterin (DC-Chol) handelt.

4. Verfahren nach einem der Ansprüche 1 bis 3, bei dem man das LPS oder das Lipid A mit dem kationischen Lipid und außerdem einem neutralen Lipid (Colipid) mischt, um einen Komplex zu bilden, in dem das LPS oder das Lipid A mit dem kationischen Lipid und dem neutralen Lipid assoziiert ist.

5. Verfahren nach Anspruch 4, wobei das neutrale Lipid aus der Gruppe bestehend aus den Folgenden ausgewählt ist: (i) Cholesterin; (ii) Phosphatidylcholinen; und (iii) Phosphatidylethanolaminen.

6. Verfahren nach Anspruch 5, wobei es sich bei dem neutralen Lipid um 1,2-Dioleoyl-*sn*-glycero-3-phosphatethanolamin (DOPE) handelt.

## Claims

1. Method for detoxification of lipopolysaccharide (LPS) or of lipid A from a Gram-negative bacterium, according to which the LPS or the lipid A is mixed with a cationic lipid so as to form a complex in which the LPS is associated with the cationic lipid,
the cationic lipid being in the form of cationic liposomes, and the LPS or the lipid A being incorporated to said liposomes, and
the cationic lipid being selected from the group constituted of:
(i) lipids incorporating an ethylphosphocholine structure; and
(ii) cationic derivatives of cholesterol.

2. Method according to Claim 1, in which the LPS is the lipooligosaccharide (LOS) from *Neisseria meningitidis.*

3. Method according to Claim 2, in which the cationic lipid is 1,2-dioleyl-sn-glycero-3-ethylphosphocholine (EDOPC) or 3β-[N-(N',N'-dimethylaminoethane)carbamoyl] cholesterol (DC-Chol).

4. Method according to one of Claims 1 to 3, according to which the LPS or the lipid A is mixed with the cationic lipid and, in addition, a neutral lipid (colipid) so as to form a complex in which the LPS or the lipid A is associated with the cationic lipid and with the neutral lipid.

5. Method according to Claim 4, in which the neutral lipid is selected from the group constituted of (i) cholesterol; (ii) phosphatidylcholines; and (iii) phosphatidylethanolamines.

6. Method according to Claim 5, in which the neutral lipid is 1,2-dioleoyl-sn-glycero-3-phosphoethanolamine (DOPE).
